# EUROPEAN PATENT APPLICATION

(11) **EP 4 365 778 A1**
(43) Date of publication of application: **08.05.2024**
(21) Application number: 23192145.3
(22) Date of filing: 18.08.2023
(51) Int. Cl.: G06N 5/025, G06N 5/046

(54) **PREDICTION PROGRAM, INFORMATION PROCESSING DEVICE, AND PREDICTION METHOD**

(30) Priority: 04.11.2022 JP 2022176995
(71) Applicant: Fujitsu Limited, Kawasaki-shi, Kanagawa 211-8588 (JP)
(72) Inventor: UKAI, Takanori, Kawasaki-shi, Kanagawa, 211-8588 (JP)
(74) Representative: Hoffmann Eitle

(57) **Abstract**

A prediction program including instructions which, when executed by a computer, cause the computer to perform processing that uses knowledge graph embedding, the processing including: determining whether or not graph data to be predicted is data that includes a node link that indicates a relationship between nodes not included in training data used for training of the knowledge graph embedding; specifying, in a case where it is determined that the graph data to be predicted is the data that includes the node link not included in the training data, graph data similar to the graph data to be predicted from the training data based on a result of embedding prediction for a label of a node included in the graph data to be predicted; and determining a prediction result for the graph data to be predicted based on the specified similar graph data.

## Description

### FIELD

The embodiment discussed herein is related to a prediction program and the like.

### BACKGROUND

A technology (referred to as "TransE") of embedding relative information including three pieces of (triple) data into knowledge graphs to perform link prediction in the knowledge graphs has been disclosed. A resource description framework (RDF) is exemplified as the relative information. The RDF indicates a data structure for describing metadata of web information and includes, as one group, three pieces of data that are an entity, a property, and an entity. Here, the entity, the property, and the entity are represented by having three elements, a subject, a predicate, and an object, as relative information. The subject, the predicate, and the object are relative information having a relation that "the predicate of the subject is the object".

In the TransE, training for the embedding of vectors of entities and properties is performed based on a set S of triple data (h, r, t) having two entities h and t belonging to E (a set of entities) and a property r belonging to R (a set of properties). In other words, the TransE is a technology of obtaining a data structure by embedding a set of knowledge graphs having three pieces of data (h, r, t) as a group (triple) into a vector space and performing vector transformation by machine learning technology. The data structure mentioned here refers to a data structure in which Vₕ + Vᵣ becomes equal to Vₜ as much as possible for respective vector representations Vₕ, Vᵣ, and Vₜ of the triple data (h, r, t).

With this configuration, using a data structure with which the TransE performs training enables calculation such as Vₕ + Vᵣ ≈ Vₜ, thus enabling prediction of t corresponding to Vₕ + Vᵣ. Furthermore, using a data structure with which the TransE performs training enables prediction of h corresponding to Vₜ - Vᵣ and r corresponding to Vₜ - Vₕ.

Furthermore, as a method of predicting whether a specific item (label of an entity) is True or False by using an embedding vector, for example, a k-nearest neighbor algorithm is exemplified. The k-nearest neighbor algorithm is a classification method based on the closest multidimensional vector in a feature space. For example, an algorithm of the k-nearest neighbor algorithm <1> determines K in advance, <2> selects K vectors closest to a new vector to be predicted, and <3> determines True or False for a specific item corresponding to the K vectors by majority rule. With this configuration, it is possible to predict whether the specific item (label of an entity) is True or False by using the new vector to be predicted.

Japanese Laid-open Patent Publication No. 2019-049980 and Antonine Bordes et al "Translating Embeddings for Modeling Multi-relational Data" are disclosed as related art.

### SUMMARY

### TECHNICAL PROBLEM

However, in a case where knowledge graph embedding is used, to predict the authenticity of a specific item for new data, it is necessary to perform re-training using the entire data including the new data again to obtain a vector of the new data. In other words, there is a problem that it takes a cost to perform the re-training using the entire data when the authenticity of the specific item is predicted for the new data.

In one aspect, an object of an embodiment is to reduce a prediction cost when the prediction of the authenticity of a specific item is performed for new data by using knowledge graph embedding.

### SOLUTION TO PROBLEM

According to an aspect of the embodiments, there is provided a prediction program including instructions which, when executed by a computer, cause the computer to perform processing that uses knowledge graph embedding, the processing including: determining whether or not graph data to be predicted is data that includes a node link that indicates a relationship between nodes not included in training data used for training of the knowledge graph embedding; specifying, in a case where it is determined that the graph data to be predicted is the data that includes the node link not included in the training data, graph data similar to the graph data to be predicted from the training data based on a result of embedding prediction for a label of a node included in the graph data to be predicted; and determining a prediction result for the graph data to be predicted based on the specified similar graph data.

### ADVANTAGEOUS EFFECTS OF INVENTION

According to one embodiment, it is possible to reduce a prediction cost when the prediction of the authenticity of a specific item is performed for new data by using knowledge graph embedding.

### BRIEF DESCRIPTION OF DRAWINGS

FIG. 1 is a functional block diagram illustrating a configuration of an information processing device according to an embodiment;
FIG. 2 is a diagram illustrating an example of training object data;
FIG. 3 is a diagram illustrating an example of new data;
FIG. 4 is a diagram illustrating an example of a knowledge graph of the training object data;
FIG. 5 is a diagram illustrating an example of a knowledge graph of the new data;
FIG. 6 is a diagram illustrating an example of training result data according to the embodiment;
FIG. 7 is a diagram illustrating a range of a training object according to the embodiment;
FIG. 8A is a diagram (1) for describing prediction processing according to the embodiment;
FIG. 8B is a diagram (2) for describing the prediction processing according to the embodiment;
FIG. 8C is a diagram (3) for describing the prediction processing according to the embodiment;
FIG. 9 is a diagram illustrating an example of a flowchart of the prediction processing according to the embodiment;
FIG. 10 is a diagram illustrating an example of a computer that executes a prediction program;
FIG. 11A is a reference diagram (1) illustrating an example of training of knowledge graph embedding;
FIG. 11B is a reference diagram (2) illustrating an example of the training of the knowledge graph embedding;
FIG. 11C is a reference diagram (3) illustrating an example of the training of the knowledge graph embedding;
FIG. 11D is a reference diagram (4) illustrating an example of the training of the knowledge graph embedding;
FIG. 12 is a reference diagram of a range of the training object in a case where a side effect of the new data is predicted; and
FIG. 13 is a reference diagram for describing the prediction of the side effect of the new data.

### DESCRIPTION OF EMBODIMENTS

Hereinafter, an embodiment of a prediction program, an information processing device, and a prediction method disclosed in the present application will be described in detail with reference to the drawings. Note that the embodiment does not limit the present disclosure.

First, "TransE" in which relative information including a plurality of pieces of data is embedded in a knowledge graph to perform link prediction of the knowledge graph will be described. The "TransE" is a technology of obtaining a predetermined data structure by embedding a set of knowledge graphs having three pieces of data as one group into a vector space and performing vector transformation by a machine learning technology. The predetermined data structure refers to a data structure in which Vₕ + Vᵣ becomes equal to Vₜ as much as possible for a vector V of each of h, r, and t in a case where one group of data is (h, r, t) (h: subject, r: predicate, t: object). With this configuration, using a data structure with which the TransE performs training enables calculation such as Vₕ + Vᵣ ≈ Vₜ, thus enabling prediction of t corresponding to Vₕ + Vᵣ. Furthermore, using a data structure with which the TransE performs training enables prediction of h corresponding to Vₜ - Vᵣ and r corresponding to Vₜ - Vₕ.

Note that data in a data format describing a relation that "the predicate of the subject is the object" with three pieces of data such as (h, r, t) as one group is referred to as resource description framework (RDF) data. Furthermore, although the RDF data has been described as a data structure having the three pieces of data of the subject, the predicate, and the object as one group, the subject and the object may be referred to as "entities" and the predicate may be referred to as "property" as appropriate.

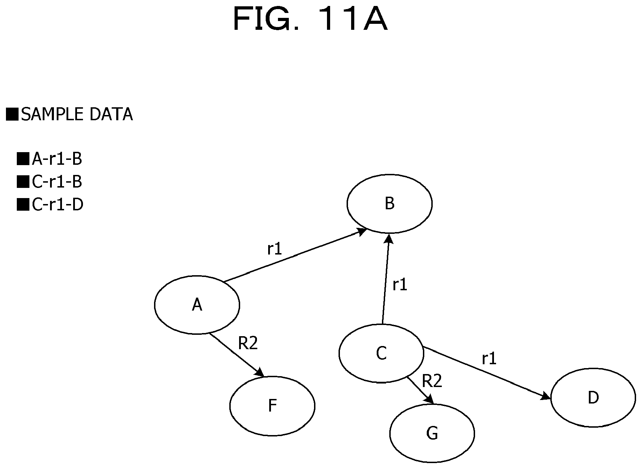

Here, an example of training of knowledge graph embedding will be described with reference to FIGs. 11A to 11D. FIGs. 11A to 11D are reference diagrams illustrating an example of the training of the knowledge graph embedding. Here, the TransE is applied as the knowledge graph embedding. Note that it is assumed that there are (A, r1, B), (C, r1, B), and (C, r1, D) as sample data of the RDF data.

A graph illustrated in FIG. 11A is a knowledge graph that represents data connectivity in the RDF data used for the training of the knowledge graph embedding. The knowledge graph embedding is a technology of mapping "A", "B", "C", and "r1" to n-dimensional vectors. In other words, in the knowledge graph embedding, mapping is performed so that "A" + "r1" becomes closer to "B", "C" + "r1" to "B", and "C" + "r1" to "D". On the other hand, mapping is performed so that "D" becomes closer to "C" + "r1" and is away from "A" + "r1". Note that, hereinafter, it is assumed that, for convenience, such training of mapping will be described in two dimensions.

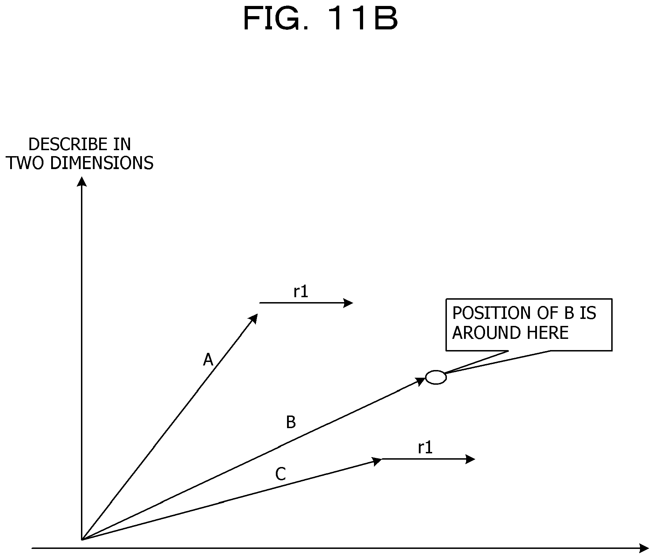

As illustrated in FIG. 11B, in the knowledge graph embedding, a vector V_{A} of "A", a vector Vᵣ₁ of "r1", a vector V_{B} of "B", and a vector V_{C} of "C" are initialized with random numbers and arranged in a two-dimensional space. Note that, although not illustrated, in the knowledge graph embedding, a vector V_{D} of "D" is also initialized with a random number and arranged in the two-dimensional space.

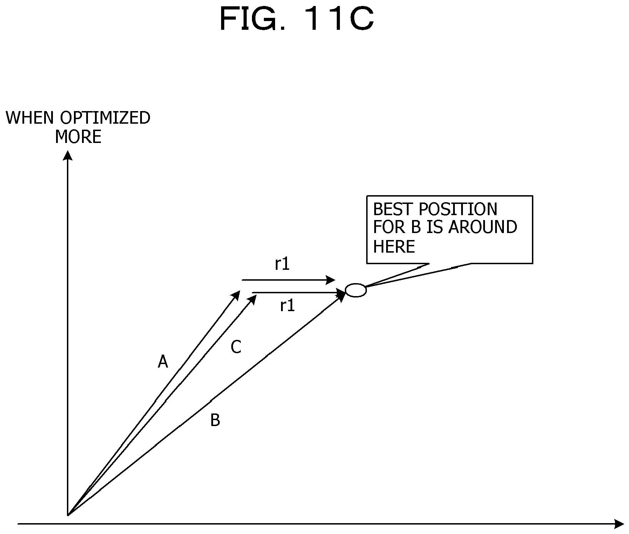

Next, as illustrated in FIG. 11C, in the knowledge graph embedding, respective vectors are optimized by training such that V_{A} + Vᵣ₁ becomes closer to V_{B} and V_{C} + Vᵣ₁ becomes closer to V_{B}. As a result, a position of B is optimized by training. In other words, training is performed such that a distance between a position indicated by V_{A} + Vᵣ₁ and a position indicated by V_{B} falls within a predetermined range (score), and a distance between a position indicated by V_{C} + Vᵣ₁ and the position indicated by V_{B} falls within a predetermined range (score).

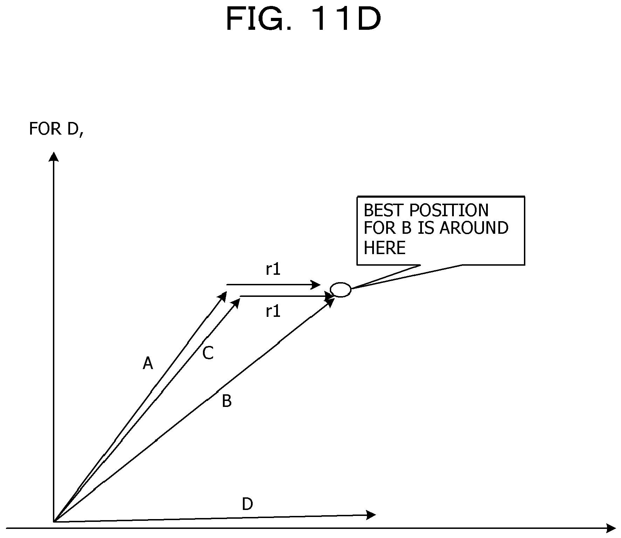

On the other hand, as illustrated in FIG. 11D, in the knowledge graph embedding, the vector V_{D} of "D" is optimized by training such that V_{C} + Vᵣ₁ becomes closer to V_{D} and V_{A} + Vᵣ₁ is away from V_{D}.

Next, it is considered that training of the knowledge graph embedding is applied to a knowledge graph in which a patient attribute, a disease, and a used medicine are set as one piece of data (case), and a side effect of new data is predicted by using vector data obtained by the application. First, for existing data, training of the knowledge graph embedding is applied in advance, and entities and a property of each piece of RDF data are mapped to n-dimensional vectors. Additionally, for the existing data, it is known whether "venous occlusion" may occur as a side effect (True) or not (False) for each case.

Here, when the new data in which the patient attribute, the disease, and the used medicine are set as one piece of data (case) is input, in training processing, training of the knowledge graph embedding is applied to knowledge graphs of the entire data including the new data and the existing data. Additionally, for the existing data, it is known whether the "venous occlusion" may occur as the side effect (True) or not (False) for each case. Thus, in the prediction processing, it is predicted whether the "venous occlusion" may occur as a side effect (True) or not (False) for the "case" of the new data by using, for example, the k-nearest neighbor algorithm for embedding vectors corresponding to the "case" of the new data and the "case" of the existing data.

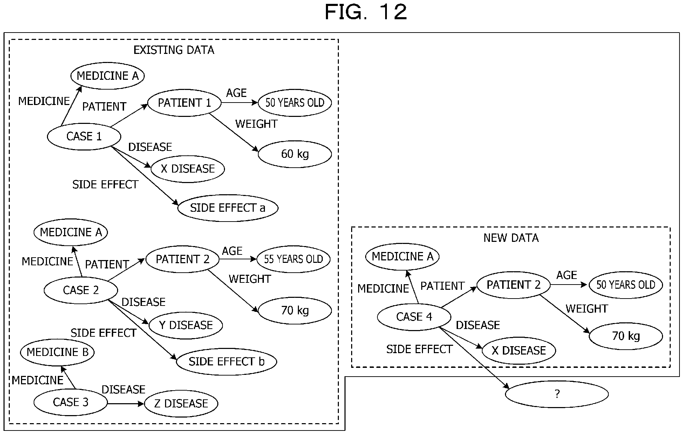

FIG. 12 is a reference diagram of a range of a training object in a case where a side effect of the new data is predicted. In a left diagram of FIG. 12, the knowledge graph of the existing data is represented. In the existing data, the knowledge graph for "Case 1", "Case 2", and "Case 3" in which a disease, a medicine, and a patient attribute are set as one case is represented. For the "Case 1" and the "Case 2", "Side effect a" and "Side effect b" are coupled as "Side effect", respectively. In a right diagram of FIG. 12, a knowledge graph of the new data is represented. In the new data, the knowledge graph for "Case 4" in which a disease, a medicine, and a patient attribute are set as one case is represented. For the "Case 4", it is unknown whether the "Side effect" may occur. Thus, in a case where the side effect of the "Case 4" is predicted, training of the knowledge graph embedding is applied to the knowledge graphs of the "Case 4" and the "Case 1" to the "Case 3" in the training processing. In other words, a portion surrounded by a solid line in FIG. 12 is the range of the training object.

As a result of the training, n-dimensional embedding vectors of entities and a property of each piece of RDF data are calculated as training data.

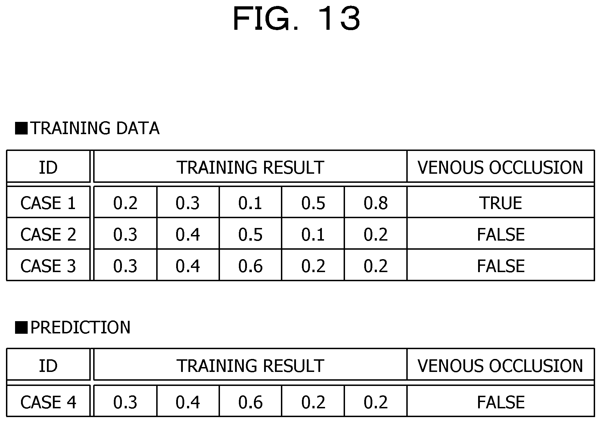

FIG. 13 is a reference diagram illustrating the prediction of the side effect of the new data. As illustrated in FIG. 13, each entity of the "Case 1", the "Case 2", the "Case 3", and the "Case 4" is calculated as a five-dimensional embedding vector. Additionally, for the "Case 1", the "Case 2", and the "Case 3", it is known whether the "venous occlusion" may occur as the side effect (True) or not (False).

In the prediction processing, it is predicted whether the "venous occlusion" may occur as the side effect (True) or not (False) for the "Case 4" as the new data by using, for example, the k-nearest neighbor algorithm for the embedding vectors in the "Case 1" to the "Case 4". For example, <1> K is determined in advance in the prediction processing. Here, it is assumed that K is determined to be "3". Then, in the prediction processing, <2> K vectors closest to the "Case 4" vector are selected. Here, since K is "3", the vectors of the "Case 1" to the "Case 3" are selected. Then, in the prediction processing, <3> True and False of the "venous occlusion" as the side effect are determined by majority rule in the K cases from "Case 1" to "Case 3". Here, one case of the "Case 1" is True, and two cases of the "Case 2" and the "Case 3" are False. Therefore, possibility of the occurrence of the "venous occlusion" as the side effect of the "Case 4" is predicted to be False as a result of the majority rule.

However, to predict whether the "venous occlusion" may occur as the side effect (True) or not (False) for the new data, it is needed to perform training using the entire data including the new data again. In other words, to predict the side effect of the new data, it is needed to perform re-training using the entirety including the new data. As a result, there is a problem that it takes a cost to perform the re-training using the entire data when the authenticity of a specific item is predicted for the new data.

Thus, in the following embodiment, an information processing device that reduces a prediction cost when the prediction of the authenticity of a specific item is performed for new data by using the knowledge graph embedding will be described.

### [Embodiment]

### [Configuration of Information Processing Device]

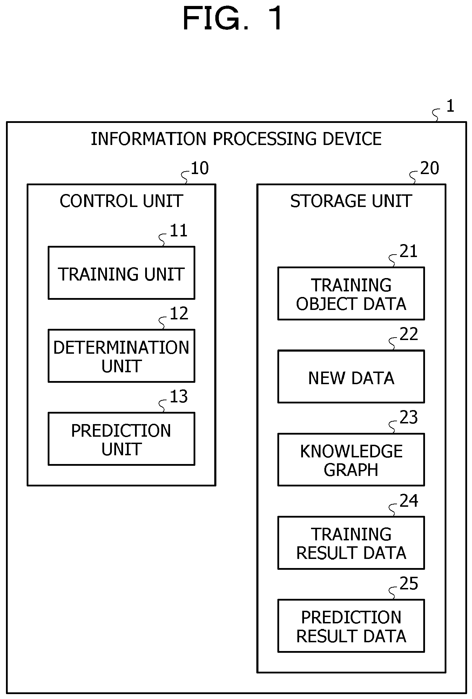

FIG. 1 is a functional block diagram illustrating a configuration of the information processing device according to the embodiment. An information processing device 1 performs link prediction for a label of a node included in graph data to be predicted by using a result of embedding prediction with which training has already been performed, and specifies graph data similar to the graph data to be predicted from training result data (training data). Then, the information processing device 1 determines a prediction result for the graph data to be predicted based on the specified graph data. Note that, in the embodiment, the information processing device 1 applies training of knowledge graph embedding to a graph data of an existing knowledge graph in which a patient attribute, a disease, and a used medicine, for which a side effect is known, are set as one piece of data (case). Then, the information processing device 1 predicts whether a side effect of the new data may be "venous occlusion" (True) or not (False) by using vector data (embedding vectors) obtained by the application.

The information processing device 1 includes a control unit 10 and a storage unit 20. The control unit 10 includes a training unit 11, a determination unit 12, and a prediction unit 13. The storage unit 20 includes training object data 21, new data 22, knowledge graphs 23, training result data 24, and prediction result data 25.

The training object data 21 is existing data indicating a training object. The training object data 21 is, for example, data (case) in which a patient attribute, a disease, a used medicine, and a side effect are associated.

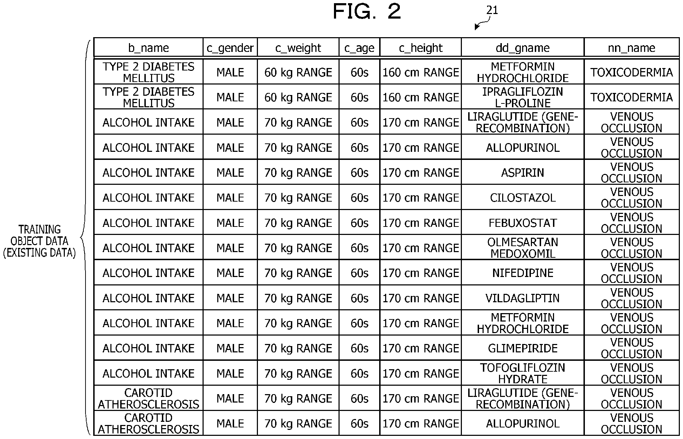

Here, an example of the training object data 21 will be described with reference to FIG. 2. FIG. 2 is a diagram illustrating an example of the training object data. As illustrated in FIG. 2, the training object data 21 is information in which b_name, c_gender, c_weight, c_age, c_height, dd_gname, and nn_name are associated as one record. The one record corresponds to one case. The b_name indicates a name of a disease. The c_gender is gender of a patient. The c_weight is a weight of a patient. The c_age is age of a patient. The c_height is a height of a patient. The c_gender, the c_weight, the c_age, and the c_height are, for example, patient attributes. The dd_gname indicates a name of a used medicine. The nn_name indicates a content of a side effect.

As an example, in the case of a case in a third record, "alcohol intake" is stored as the b_name, "male" is stored as the c_gender, "70 kg range" is stored as the c_weight, "60s" is stored as the c_age, and "170 cm range" is stored as the c_height. Moreover, "liraglutide (gene-recombination)" is stored as the dd_gname, and "venous occlusion" is stored as the side effect.

Returning to FIG. 1, the new data 22 is new data to be predicted. For example, the new data 22 is data (case) in which a patient attribute, a disease, and a used medicine are associated. In other words, the new data 22 is data for which whether or not a side effect may be "venous occlusion" is to be predicted.

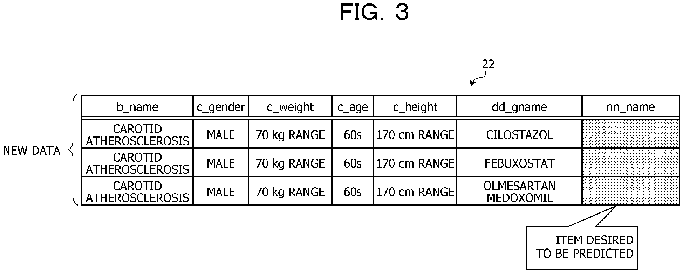

Here, an example of the new data 22 will be described with reference to FIG. 3. FIG. 3 is a diagram illustrating an example of the new data. As illustrated in FIG. 3, the new data 22 is information in which b_name, c_gender, c_weight, c_age, c_height, dd_gname, and nn_name are associated as one record. The one record corresponds to one case. Since the respective items are the same as those of the training object data 21 in FIG. 2, description thereof will be omitted. In the new data 22, a content of the nn_name indicating a name of a side effect is blank. The content of the nn_name is an item desired to be predicted.

Returning to FIG. 1, the knowledge graphs 23 are information obtained by converting each training object data 21 and the new data 22 into graph data along a predetermined entity-relationship (ER) diagram. The conversion for each case obtains the graph data. Additionally, the graph data includes a plurality of pieces of RDF data having three pieces of data of an entity, a property, and an entity as one group for one case. Note that the ER diagram is a diagram in which a relationship between an entity and an entity is designed.

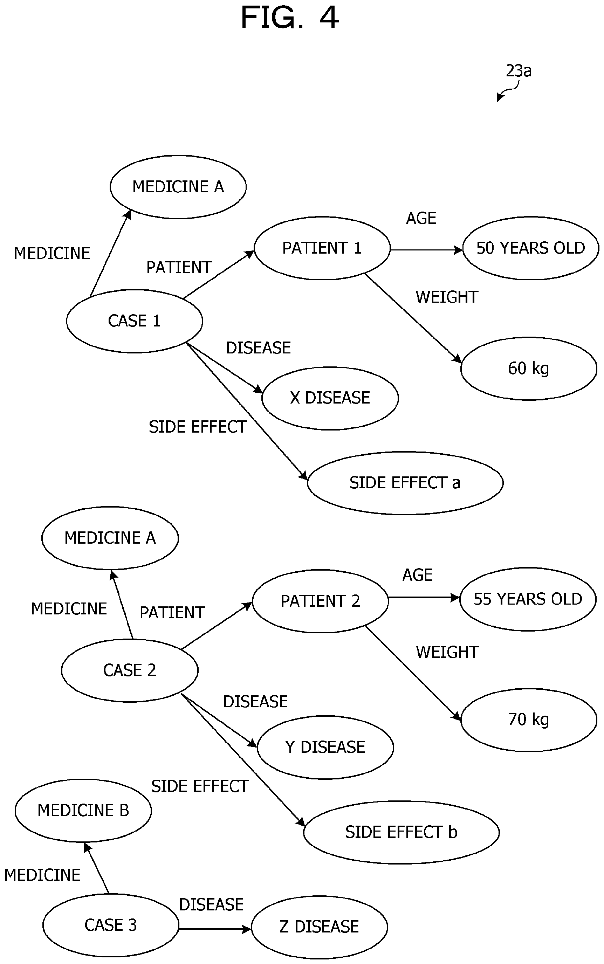

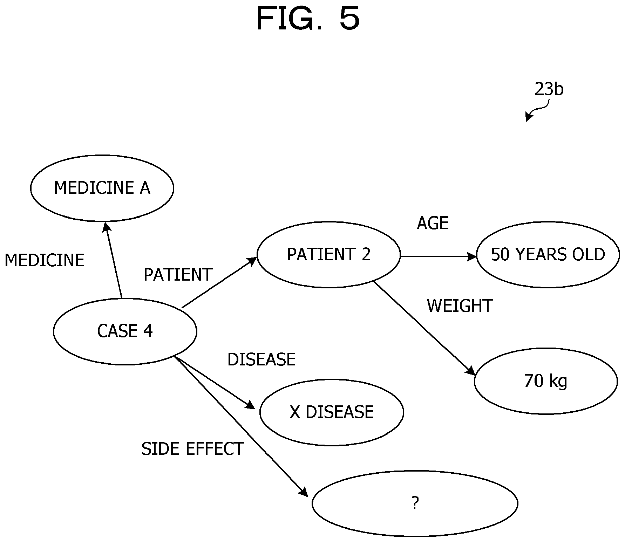

Here, an example of a knowledge graph of each of the training object data 21 and the new data 22 will be described with reference to FIGs. 4 and 5.

FIG. 4 is a diagram illustrating an example of the training object data knowledge graph. As illustrated in FIG. 4, each piece of graph data included in a knowledge graph 23a is obtained by converting each record (case) of the training object data 21. For example, "Case 1" includes a plurality of pieces of RDF data having three pieces of data as one group, such as "Case 1 → Medicine → Medicine A", "Case 1 → Patient → Patient 1", "Case 1 → Disease → X disease", and "Case 1 → Side effect → Side effect a", for example. For example, in the "Case 1 → Medicine → Medicine A", the "Case 1" and the "Medicine A" are entities, the "Medicine" is a property, and there is a relation that "the medicine in the Case 1 is the Medicine A". In the "Case 1 → Side effect → Side effect a", the "Case 1" and the "Side effect a" are entities, the "Side effect" is a property, and there is a relation that "the side effect in the Case 1 is the Side effect a". Note that, for "Case 3", RDF data of the patient attribute and RDF data of the side effect are not included, but such a case is also converted as a knowledge graph.

FIG. 5 is a diagram illustrating an example of the knowledge graph of the new data. As illustrated in FIG. 5, graph data indicated by a knowledge graph 23b is obtained by converting a record (case) of the new data 22. For example, "Case 4" as the new data 22 also includes a plurality of pieces of RDF data having three pieces of data as one group, such as "Case 4 → Medicine → Medicine A", "Case 4 → Patient → Patient 2", and "Case 4 → Disease → X disease", for example. However, for the "Case 4", "Side effect" of the "Case 4" is unknown.

Returning to FIG. 1, the training result data 24 is information indicating a result of applying training of knowledge graph embedding by using RDF data included in a plurality of pieces of graph data obtained by converting the training object data 21. The training result data 24 is information in which an n-dimensional embedding vector mathematically represented by the training of the knowledge graph embedding is associated with each of entities and properties constituting the plurality of pieces of graph data obtained by converting the training object data 21. Note that there is a case where labels of the entities are the same among the plurality of pieces of graph data. In this case, the labels of the entities have the same vector value. Similarly, there is a case where labels of the properties are the same among the plurality of pieces of graph data. In this case, the labels of the properties have the same vector value.

In addition, in the training result data 24, information regarding a side effect is associated with each piece of graph data. The information regarding a side effect is, for example, information indicating whether "venous occlusion" may occur as a side effect (True) or not (False). Note that the training result data 24 is generated by the training unit 11 to be described later. An example of the training result data 24 will be described later.

The prediction result data 25 is information indicating a result of predicting whether "venous occlusion" may occur as a side effect (True) or not (False) for graph data obtained by converting the new data 22. Note that the prediction result data 25 is generated by the prediction unit 13 to be described later.

The training unit 11 applies training of knowledge graph embedding by using RDF data included in a plurality of pieces of graph data obtained by converting the training object data 21. In other words, the training unit 11 generates an n-dimensional embedding vector by applying the training of the knowledge graph embedding for each of entities and properties constituting the plurality of pieces of graph data obtained by converting the training object data 21. Then, the training unit 11 stores a training result in the training result data 24. In other words, the training unit 11 stores the n-dimensional embedding vector indicating a result of training in the training result data 24 for each of the entities and the properties constituting the plurality of pieces of graph data as a training object. In addition, the training unit 11 stores, in the training result data 24, information regarding a side effect in association with a head node (entity) of the graph data as the training object. The information regarding a side effect is, for example, information indicating whether "venous occlusion" may occur as a side effect (True) or not (False). It is sufficient that the information regarding a side effect is associated with the content of the nn_name for each case in the training object data 21. Note that a method of training the knowledge graph embedding has been described with reference to FIGs. 11A to 11D, and thus description thereof will be omitted. Furthermore, the training of knowledge graph embedding may be referred to as "learning of knowledge graph embedding", which may correspond to training a neural network model with respect to knowledge graph embedding.

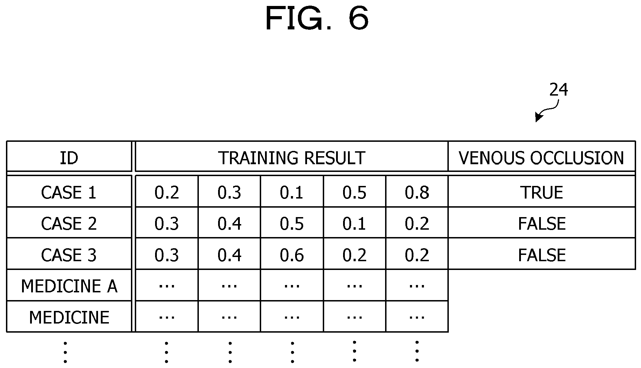

Here, an example of the training result data 24 will be described with reference to FIG. 6. FIG. 6 is a diagram illustrating an example of the training result data according to the embodiment. As illustrated in FIG. 6, the training result data 24 stores an identifier (ID), a training result, and venous occlusion in association with each other. The ID indicates labels of entities and properties constituting graph data as a training object. The content for the training result indicates a five-dimensional embedding vector. Note that the content for the training result is not limited to the five-dimensional embedding vector, and it is sufficient that the content is an n-dimensional embedding vector (n is 2 or more).

Furthermore, the content for the venous occlusion is associated with "Case" indicating a head node (entity) of the graph data, and indicates whether "venous occlusion" may occur as a side effect (True) or not (False). It is sufficient that the content for the venous occlusion is associated from the content (nn_name) of the side effect for each case stored in the training object data 21.

As an example, in a case where the ID is "Case 1", (0.2, 0.3, 0.1, 0.5, and 0.8) is stored as the training result, and "TRUE" is stored as the venous occlusion. Furthermore, in a case where the ID is "Medicine A", a five-dimensional embedding vector is stored as the training result.

The determination unit 12 determines whether or not graph data to be predicted is data including a node link indicating a relationship between nodes not included in the training object data 21 used for training of knowledge graph embedding. In a case where the graph data to be predicted is the data including the node link indicating the relationship between the nodes not included in the training object data 21, the determination unit 12 sets the graph data to be predicted as new data.

Here, the new data will be described. The new data refers to data indicating a new case not included in the training object data 21. Note that, although the new data is data indicating a new case not included in training data, but is not data indicating a completely new case. For example, the new data is data of a case where a medicine different from one that has been previously administered is administered to the same patient with the same disease, or data of a case where an existing medicine is administered to a patient with the same disease but a different patient attribute. That is, the new data means data in which any one of the disease, the patient attribute, and the medicine is new, and data in which all of the disease, the patient attribute, and the medicine are new is excluded from the new data.

Specifically, in a case where there is the graph data of the training object data 21 illustrated in FIG. 4, the graph data illustrated in FIG. 5 is data indicating a new case not included in the training object data 21. That is, the "Case 4" of the graph data illustrated in FIG. 5 is a case where the "Medicine A" is administered as the medicine when the disease is the "X disease" for the "Patient 2" having the patient attributes of the age "50 years old" and the weight "70 kg". In the training object data 21, there is a case where the "Medicine A" is administered for "Y disease" for the "Patient 2" in the "Case 2", but there is no case where the "Medicine A" is administered for the "X disease". Furthermore, in the training object data 21, there is a case where the "Medicine A" is administered for the "X disease" in the "Case 1", but the patient attributes of the "Case 1" are different from those of the "Case 4". In other words, the "Patient 1" in the "Case 1" is a patient having the patient attributes of the age "50 years old" and the weight "60 kg", but the patient attributes are different from the patient attributes (Age → "50 years old", Weight → "60 kg") of the "Patient 2" in the "Case 4". Therefore, the graph data of the "Case 4" is a new case that is not included in the training object data 21, and is thus the new data.

Returning to FIG. 1, for example, the determination unit 12 determines whether or not the graph data to be predicted is included in any graph data of the training object data 21 (existing data). As an example, the determination unit 12 determines whether or not graph data in which labels of nodes other than a head node of the graph data to be predicted exactly match exists in the graph data of the existing data. Specifically, a case where the graph data to be predicted is the graph data illustrated in FIG. 5 and where the graph data as the existing data is the graph data illustrated in FIG. 4 will be described. In the graph data to be predicted, the medicine is the "Medicine A", the age is "50 years old", the weight is "70 kg", and the disease is the "X disease" for the patient, and the determination unit 12 determines whether or not graph data that exactly matches these exists in the graph data of the existing data. Since the graph data that exactly matches these does not exist in the three pieces of graph data of the existing data, the determination unit 12 sets the graph data to be predicted as the new data.

The prediction unit 13 specifies, in a case where it is determined that graph data to be predicted is new data, graph data similar to the graph data to be predicted from the training object data 21 based on a result of embedding prediction for a label of a node included in the graph data to be predicted.

For example, the prediction unit 13 searches for a label of a known entity and a label of a known property included in the graph data to be predicted with reference to a knowledge graph of the training object data 21. Then, the prediction unit 13 calculates a score (distance) by performing link prediction with a plurality of pieces of graph data (cases) included in the training object data 21 by using embedding vectors that have already been used for training for the labels of the known entity and property that may be searched for. It is sufficient that the embedding vectors that have already been used for training are acquired from the training result data 24. Note that, in a case where there is a plurality of labels of the known entities included in the graph data to be predicted, it is sufficient for the prediction unit 13 to calculate the scores (distances) from the plurality of pieces of graph data included in the training object data 21 for the plurality of labels of the known entities.

As an example, a score calculation method in a case where graph data to be predicted "4" includes "Medicine A" as the label of the known entity and "Medicine" as the label of the known property will be described. For graph data included in the knowledge graph of the training object data 21, embedding vectors are associated with labels of constituent entities and properties and stored in the training result data 24. Additionally, for the known "Medicine A" and "Medicine", embedding vectors that have already been used for training are stored in the training result data 24. Thus, the prediction unit 13 calculates the score (distance) by performing link prediction between the label "Medicine A" of the known entity and a label of a head node of the known graph data. In a case where the label of the head node of the known graph data is "Case 1", the score (distance) is calculated by a calculation expression of |"Medicine A" - "Medicine" - "Case 1"|. In the training of the knowledge graph embedding, in a case where RDF data is (A, r1, B), mapping is performed so that "A" + "r1" becomes closer to "B". Thus, the prediction unit 13 may calculate the score (distance) between the "Medicine A" and the "Case 1" by calculating an absolute value of ("Medicine A" - "Medicine" - "Case 1").

Then, the prediction unit 13 ranks the labels of the known entity in ascending order of the score (distance) for each of the plurality of pieces of graph data. Then, the prediction unit 13 multiplies the rank by the score for each of the plurality of pieces of graph data to give a rank score. The rank score is given in order to perform modulation so that the graph data with a short distance (with a small score) becomes closer (smaller). Then, the prediction unit 13 specifies graph data having the smallest rank score from the plurality of pieces of graph data as graph data similar to the graph data to be predicted.

Note that, in the case where there is the plurality of labels of the known entities, the prediction unit 13 gives a rank score to each label of the known entity for each of the plurality of pieces of graph data. Then, the prediction unit 13 calculates the sum of the rank scores for each of the plurality of pieces of graph data, and gives a total score. Then, it is sufficient for the prediction unit 13 to specify graph data having the smallest total score from the plurality of pieces of graph data as graph data similar to the graph data to be predicted.

Then, the prediction unit 13 determines a prediction result for the graph data to be predicted based on the specified similar graph data. As an example, the prediction unit 13 refers to the training object data 21 to acquire a content (nn_name) of a side effect of a record (case) corresponding to the specified similar graph data. Then, when the acquired content of the side effect is "venous occlusion", the prediction unit 13 determines "True" by determining that the "venous occlusion" may occur as a prediction result for the graph data to be predicted. Furthermore, when the acquired content of the side effect is not the "venous occlusion", the prediction unit 13 determines "False" by determining that the "venous occlusion" may not occur as a prediction result for the graph data to be predicted.

As another example, the prediction unit 13 refers to the training result data 24 to acquire a result of predicting "venous occlusion" for the specified similar graph data. Then, the prediction unit 13 determines the acquired result of predicting "venous occlusion" (True or False) as a prediction result for the graph data to be predicted.

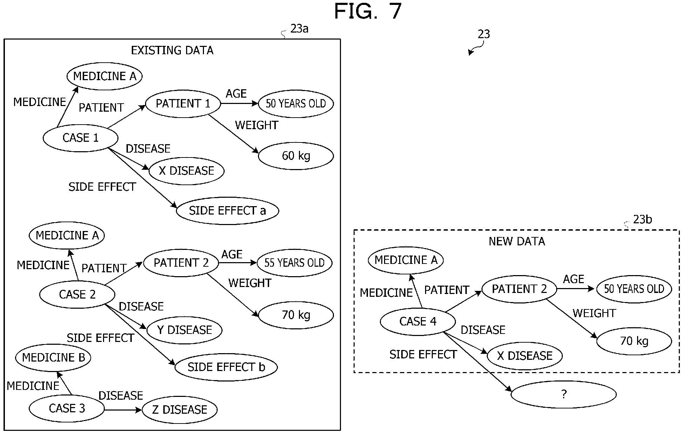

FIG. 7 is a diagram illustrating a range of a training object according to the embodiment. In FIG. 7, the knowledge graphs 23 of the existing data (training object data 21) and the new data are represented. In a left diagram of FIG. 7, the knowledge graph 23a of the existing data (training object data 21) is represented. In the existing data, the knowledge graph for "Case 1", "Case 2", and "Case 3" in which a disease, a medicine, and a patient attribute are set as one case is represented. For the "Case 1" and the "Case 2", "Side effect a" and "Side effect b" are coupled as "Side effect", respectively. In a right diagram of FIG. 7, the knowledge graph 23b of the new data is represented. In the new data, the knowledge graph for "Case 4" in which a disease, a medicine, and a patient attribute are set as one case is represented. For the "Case 4", it is unknown whether the "Side effect" may occur.

Under such a situation, in the embodiment, the training unit 11 applies training of knowledge graph embedding to the knowledge graph 23a of the "Case 1" to the "Case 3" indicating the existing data. In other words, the range of the training object according to the embodiment does not target the new data but targets only the existing data surrounded by a solid line in FIG. 7. With this configuration, when predicting whether the new data is True or False for a specific item, the training unit 11 does not need to perform re-training including the new data, so that a prediction cost may be reduced.

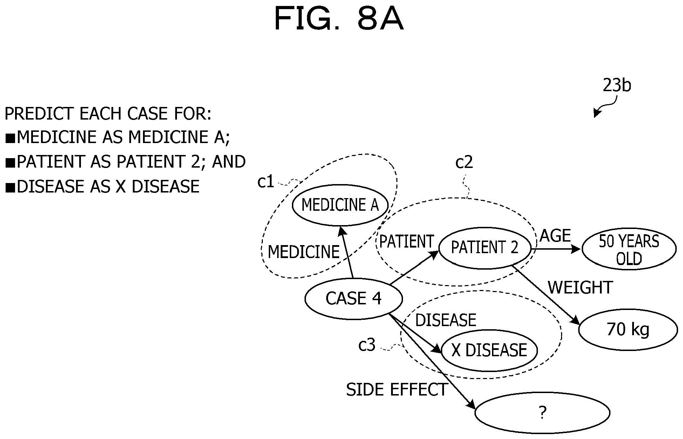

FIGs. 8A to 8C are diagrams for describing the prediction processing according to the embodiment.

In FIG. 8A, the knowledge graph 23b of the new data is represented. Graph data of the new data used here is the same as the graph data illustrated in the right diagram of FIG. 7. Furthermore, although not illustrated, it is assumed that a plurality of pieces of graph data of the existing data used here is the same as the graph data illustrated in the left diagram of FIG. 7.

Under such a situation, the prediction unit 13 searches for a label of a known entity and a label of a known property included in graph data to be predicted with reference to a knowledge graph of the training object data 21. Here, "Medicine A" is searched for as the label of the known entity, and "Medicine" is searched for as the label of the known property (reference sign c1). Furthermore, "Patient 2" is searched for as the label of the known entity, and "Patient" is searched for as the label of the known property (reference sign c2). Furthermore, "X disease" is searched for as the label of the known entity, and "Disease" is searched for as the label of the known property (reference sign c3). Thus, the prediction unit 13 predicts scores (distances) from each case included in the existing data for the "Medicine" as the "Medicine A", the "Patient" as the "Patient 2", and the "Disease" as the "X disease".

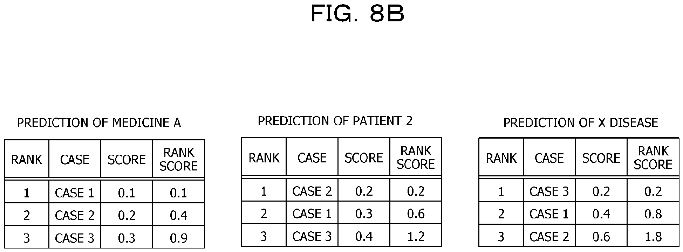

As illustrated in FIG. 8B, the prediction unit 13 uses embedding vectors that have already been used for training for the labels of the known entity and property included in the graph data to be predicted. Then, the prediction unit 13 calculates scores (distances) between the label of the known entity and a plurality of cases included in the existing data by performing link prediction.

Here, the prediction unit 13 calculates a score (distance) between the label "Medicine A" of the known entity and "Case 1". The score (distance) between the "Medicine A" and the "Case 1" is calculated by the calculation expression of |"Medicine A" - "Medicine" - "Case 1"|. Similarly, a score (distance) between the "Medicine A" and "Case 2" is calculated by a calculation expression of |"Medicine A" - "Medicine" - "Case 2"|. A score (distance) between the "Medicine A" and "Case 3" is calculated by a calculation expression of |"Medicine A" - "Medicine" - "Case 3"|. For the "Medicine A", the scores are represented as in a left table in FIG. 8B for each case. The score is calculated as "0.1" in a case where the case is the "Case 1", the score is calculated as "0.2" in a case where the case is the "Case 2", and the score is calculated as "0.3" in a case where the case is the "Case 3". Therefore, ranks are determined in the order of the "Case 1", the "Case 2", and the "Case 3". Moreover, a rank score is obtained by multiplying the rank by the score. Therefore, the rank score is calculated as "0.1" in a case where the case is the "Case 1", the rank score is calculated as "0.4" in a case where the case is the "Case 2", and the rank score is calculated as "0.9" in a case where the case is the "Case 3".

Similarly, the prediction unit 13 calculates a score (distance) between the label "Patient 2" of the known entity and the "Case 1". The score (distance) between the "Patient 2" and the "Case 1" is calculated by a calculation expression of |"Patient 2" - "Patient" - "Case 1"|. Similarly, a score (distance) between the "Patient 2" and the "Case 2" is calculated by a calculation expression of |"Patient 2" - "Patient" - "Case 2"|. A score (distance) between the "Patient 2" and the "Case 3" is calculated by a calculation expression of |"Patient 2" - "Patient" - "Case 3"|. For the "Patient 2", the scores are represented as in a middle table in FIG. 8B for each case. The score is calculated as "0.3" in a case where the case is the "Case 1", the score is calculated as "0.2" in a case where the case is the "Case 2", and the score is calculated as "0.4" in a case where the case is the "Case 3". Therefore, ranks are determined in the order of the "Case 2", the "Case 1", and the "Case 3". Moreover, a rank score is obtained by multiplying the rank by the score. Therefore, the rank score is calculated as "0.6" in a case where the case is the "Case 1", the rank score is calculated as "0.2" in a case where the case is the "Case 2", and the rank score is calculated as "1.2" in a case where the case is the "Case 3".

Similarly, the prediction unit 13 calculates a score (distance) between the label "X disease" of the known entity and the "Case 1". The score (distance) between the "X disease" and the "Case 1" is calculated by a calculation expression of |"X disease" - "Disease" - "Case 1"|. Similarly, a score (distance) between the "X disease" and the "Case 2" is calculated by a calculation expression of |"X disease" - "Disease" - "Case 2"|. A score (distance) between the "X disease" and the "Case 3" is calculated by a calculation expression of |"X disease" - "Disease" - "Case 3"|. For the "X disease", the scores are represented as in a right table in FIG. 8B for each case. The score is calculated as "0.4" in a case where the case is the "Case 1", the score is calculated as "0.6" in a case where the case is the "Case 2", and the score is calculated as "0.2" in a case where the case is the "Case 3". Therefore, ranks are determined in the order of the "Case 3", the "Case 1", and the "Case 2". Moreover, a rank score is obtained by multiplying the rank by the score. Therefore, the rank score is calculated as "0.8" in a case where the case is the "Case 1", the rank score is calculated as "1.8" in a case where the case is the "Case 2", and the rank score is calculated as "0.2" in a case where the case is the "Case 3".

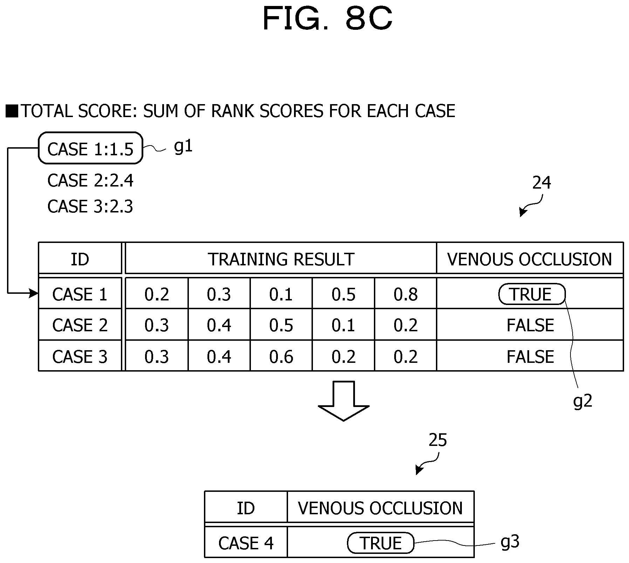

As illustrated in FIG. 8C, the prediction unit 13 calculates the sum of the rank scores for each of the plurality of graph data (cases) pieces, and gives a total score. Here, for the "Case 1", the prediction unit 13 calculates a total score "1.5" by summing the rank score "0.1" for the "Medicine A", the rank score "0.6" for the "Patient 2", and the rank score "0.8" for the "X disease". For the "Case 2", the prediction unit 13 calculates a total score "2.4" by summing the rank score "0.4" for the "Medicine A", the rank score "0.2" for the "Patient 2", and the rank score "1.8" for the "X disease". For the "Case 3", the prediction unit 13 calculates a total score "2.3" by summing the rank score "0.9" for the "Medicine A", the rank score "1.2" for the "Patient 2", and the rank score "0.2" for the "X disease".

Then, the prediction unit 13 specifies graph data (case) having the smallest total score from the plurality of pieces of graph data (cases) as graph data (case) similar to the graph data to be predicted (Case 4). Here, the case having the smallest total score is the "Case 1". Therefore, the prediction unit 13 specifies the "Case 1" as a case similar to the "Case 4" to be predicted (reference sign g1).

Then, the prediction unit 13 refers to the training result data 24 to acquire a result of predicting "venous occlusion" for the specified similar graph data (case). Here, the specified similar case is the "Case 1". Thus, the prediction unit 13 acquires "TRUE" as a result of predicting the "venous occlusion" for the "Case 1" (reference sign g2).

Then, the prediction unit 13 determines the acquired "TRUE" indicating the result of predicting the "venous occlusion" as a prediction result for the "Case 4" to be predicted, and stores the prediction result in the prediction result data 25 (reference sign g3). With this configuration, the prediction unit 13 specifies the existing "case" similar to the "Case 4" by using the embedding vectors of the items with which training has already been performed for the new data (Case 4), and predicts possibility of occurrence of the "venous occlusion". As a result, the prediction unit 13 may reduce a prediction cost when predicting the possibility of the occurrence of the "venous occlusion" for the new data (Case 4).

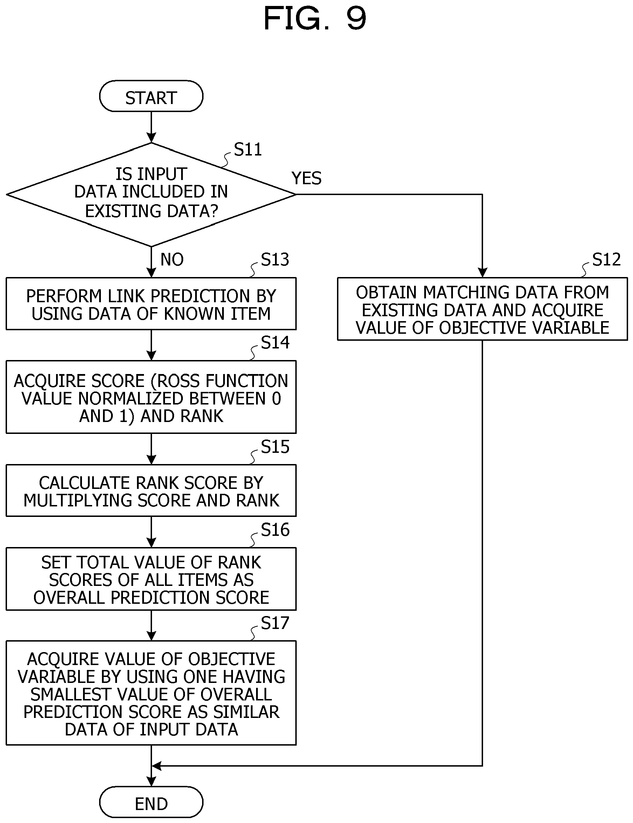

FIG. 9 is a diagram illustrating an example of a flowchart of the prediction processing according to the embodiment. Note that it is assumed that the training unit 11 applies training of knowledge graph embedding by using RDF data included in a plurality of graph data obtained by converting the training object data 21, and stores a training result in the training result data 24. Then, it is assumed that input data indicating graph data to be predicted is received.

As illustrated in FIG. 9, the determination unit 12 determines whether or not the received input data is included in the existing data (Step S11). For example, the determination unit 12 determines whether or not the graph data to be predicted is included in any graph data of the training object data 21 (existing data). As an example, the determination unit 12 determines whether or not graph data in which labels of nodes other than a head node of the graph data to be predicted exactly match exists in the graph data of the existing data.

In a case where it is determined that the input data is included in the existing data (Step S11; Yes), the determination unit 12 obtains matching data from the existing data and acquires a value of an objective variable (Step S12). The objective variable mentioned here refers to, for example, "venous occlusion", and the value of the objective variable refers to, for example, "False" or "True". For example, the determination unit 12 refers to the training result data 24 to acquire a content of the "venous occlusion" corresponding to a case indicated by the graph data that exactly matches. Then, the determination unit 12 ends the prediction processing.

On the other hand, when it is determined that the input data is not included in the existing data (Step S11; No), the prediction unit 13 performs link prediction by using data of a known item (Step S13). For example, the prediction unit 13 searches for a label of a known entity and a label of a known property included in the graph data to be predicted with reference to a knowledge graph of the training object data 21. Then, the prediction unit 13 calculates a score (distance) by performing link prediction with a plurality of pieces of graph data (cases) included in the training object data 21 by using embedding vectors that have already been used for training for the labels of the known entity and property that may be searched for.

Then, the prediction unit 13 acquires a score and a rank for each piece of the existing data for the known item (Step S14). For example, the prediction unit 13 acquires a rank and a score (distance) between the label of the known entity and each piece of graph data (case) included in the knowledge graph of the training object data 21. Note that, in a case where there is a plurality of labels of the known entities, the prediction unit 13 acquires a rank and a score (distance) from each piece of graph data (case) for each label.

Then, the prediction unit 13 calculates a rank score by multiplying the score and the rank for each piece of the known data for the known item (Step S15). Then, the prediction unit 13 sets a total value of the rank scores of all the known items as an overall prediction score (total score) for each piece of the known data (Step S16).

Then, the prediction unit 13 acquires a value of an objective variable by using known data having the smallest value of the overall prediction score (total score) as similar data of the input data (Step S17). In other words, the prediction unit 13 specifies graph data having the smallest total score from the plurality of pieces of graph data as graph data similar to the graph data to be predicted. Then, the prediction unit 13 refers to the training result data 24 to acquire a result of predicting "venous occlusion" for the specified similar graph data. Then, the prediction unit 13 determines the acquired result of predicting "venous occlusion" (True or False) as a prediction result for the graph data to be predicted. Then, the determination unit 12 ends the prediction processing.

### [Effects of Embodiment]

According to the embodiment described above, the information processing device 1 determines whether or not graph data to be predicted is data including a node link indicating a relationship between nodes not included in training data used for training of knowledge graph embedding. The information processing device 1 specifies, in a case where it is determined that the graph data to be predicted is the data including the node link not included in the training data, graph data similar to the graph data to be predicted from the training data based on a result of embedding prediction for a label of a node included in the graph data to be predicted. Then, the information processing device 1 determines a prediction result for the graph data to be predicted based on the specified similar graph data. According to such a configuration, in the knowledge graph embedding, the information processing device 1 may reduce a prediction cost when predicting authenticity of True or False of a specific item for the graph data to be predicted. In other words, since the information processing device 1 may predict the prediction result without performing re-training including the graph data to be predicted, the prediction cost may be reduced.

Furthermore, according to the embodiment described above, the information processing device 1 calculates similarity between the label of the node and each piece of graph data by performing link prediction with a plurality of pieces of graph data included in the training data by using an embedding vector of the label of the node that has already been used for training for the label of the node included in the graph data to be predicted. Then, the information processing device 1 specifies the most similar graph data from the plurality of pieces of graph data as graph data similar to the graph data to be predicted. According to such a configuration, the information processing device 1 calculates the similarity between the label of the node included in the graph data to be predicted and the plurality of pieces of graph data included in the training data by using the link prediction, and specifies the graph data similar to the graph data to be predicted. As a result, the information processing device 1 may specify the graph data similar to the graph data to be predicted from the plurality of pieces of graph data used for training by using the link prediction, and may reduce the prediction cost by using the specified graph data for prediction.

Furthermore, according to the embodiment described above, the similarity between the label of the node and the graph data is a distance between the label of the node and the graph data. The label of the node included in the graph data to be predicted is the same label as the label of the node that has already been used for training included in the training data. In a case where there is a plurality of labels of the nodes included in the graph data to be predicted, the information processing device 1 specifies graph data having the smallest distance from the plurality of pieces of graph data by using the distance between each label of the plurality of nodes and each of the plurality of pieces of graph data. According to such a configuration, the information processing device 1 may accurately specify the graph data similar to the graph data to be predicted by using the plurality of labels of the node included in the graph data to be predicted.

Furthermore, according to the embodiment described above, the information processing device 1 specifies graph data having the smallest total value of the distances from the plurality of pieces of graph data by using a total value of the distances from the respective labels of the plurality of nodes for each of the plurality of pieces of graph data. According to such a configuration, the information processing device 1 may accurately specify the graph data similar to the graph data to be predicted by using the plurality of labels of the node included in the graph data to be predicted.

Furthermore, according to the embodiment described above, the information processing device 1 determines a value corresponding to a label of a specific node included in the similar graph data as a prediction result for the graph data to be predicted. According to such a configuration, for example, in a case where the label of the specific node included in the similar graph data is "venous occlusion" indicating a side effect, the information processing device 1 may determine a value indicating whether the "venous occlusion" may occur as a side effect or not as a prediction result.

### [Others]

Note that each illustrated component of the information processing device 1 does not necessarily have to be physically configured as illustrated in the drawings. In other words, specific aspects of separation and integration of the information processing device 1 are not limited to the illustrated ones, and all or a part thereof may be functionally or physically separated or integrated in any unit depending on various loads, use situations, and the like. For example, the determination unit 12 and the prediction unit 13 may be integrated as one unit. Furthermore, the storage unit 20 may be coupled through a network as an external device of the information processing device 1.

Furthermore, in the embodiment described above, the configuration in which the information processing device includes the training and prediction processing has been described. However, the configuration in which a training device that performs the training processing and a prediction device that performs the prediction processing are separated may be adopted.

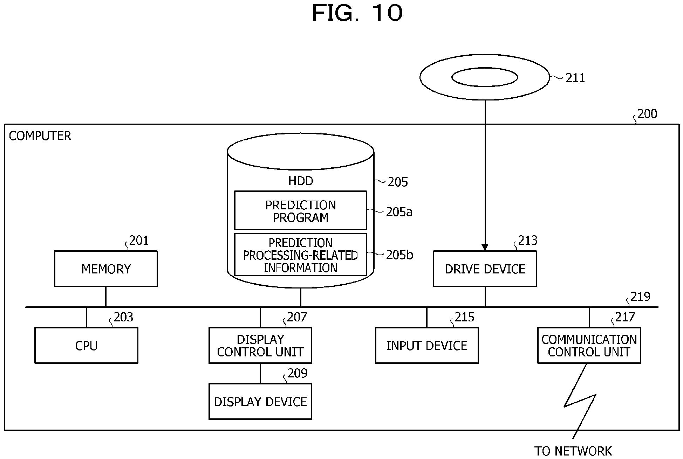

Furthermore, various types of the processing described in the embodiment described above may be implemented by a computer such as a personal computer or a workstation executing a program prepared in advance. Thus, in the following, an example of a computer that executes a prediction program that implements functions similar to those of the prediction processing of the information processing device 1 illustrated in FIG. 1 will be described. FIG. 10 is a diagram illustrating an example of the computer that executes the prediction program.

As illustrated in FIG. 10, a computer 200 includes a central processing unit (CPU) 203 that executes various types of arithmetic processing, an input device 215 that receives data input from a user, and a display control unit 207 that controls a display device 209. Furthermore, the computer 200 includes a drive device 213 that reads a program and the like from a storage medium, and a communication control unit 217 that exchanges data with another computer via a network. Furthermore, the computer 200 includes a memory 201 that temporarily stores various types of information, and a hard disk drive (HDD) 205. Additionally, the memory 201, the CPU 203, the HDD 205, the display control unit 207, the drive device 213, the input device 215, and the communication control unit 217 are coupled by a bus 219.

The drive device 213 is, for example, a device for a removable disk 211. The HDD 205 stores a prediction program 205a and prediction processing-related information 205b.

The CPU 203 reads the prediction program 205a to load the read prediction program 205a into the memory 201, and executes the loaded prediction program 205a as a process. Such a process corresponds to each functional unit of the information processing device 1. The prediction processing-related information 205b corresponds to the training object data 21, the new data 22, the knowledge graph 23, the training result data 24, and the prediction result data 25. Additionally, for example, the removable disk 211 stores each piece of information such as the prediction program 205a.

Note that the prediction program 205a does not necessarily have to be stored in the HDD 205 from the beginning. For example, the program is stored in a "portable physical medium" to be inserted into the computer 200, such as a flexible disk (FD), a compact disk read only memory (CD-ROM), a digital versatile disk (DVD), a magneto-optical disk, or an integrated circuit (IC) card. Then, the computer 200 may read the prediction program 205a from these and execute the read prediction program 205a.

## Claims

1. A prediction program comprising instructions which, when executed by a computer, cause the computer to perform processing that uses knowledge graph embedding, the processing comprising:
determining whether or not graph data to be predicted is data that includes a node link that indicates a relationship between nodes not included in training data used for training of the knowledge graph embedding;
specifying, in a case where it is determined that the graph data to be predicted is the data that includes the node link not included in the training data, graph data similar to the graph data to be predicted from the training data based on a result of embedding prediction for a label of a node included in the graph data to be predicted; and
determining a prediction result for the graph data to be predicted based on the specified similar graph data.

2. The prediction program according to claim 1, wherein
the specifying includes:
calculating similarity between the label of the node and each piece of graph data, by performing link prediction with a plurality of pieces of graph data included in the training data by using an embedding vector of a label of a node that has already been used for training for the label of the node included in the graph data to be predicted; and
specifying, as graph data similar to the graph data to be predicted, graph data most similar from among the plurality of pieces of graph data.

3. The prediction program according to claim 2, wherein
the similarity between the label of the node and the graph data is a distance between the label of the node and the graph data,
the label of the node included in the graph data to be predicted is the same label as the label of the node that has already been used for training included in the training data, and
the specifying includes
in a case where there is a plurality of labels of the nodes included in the graph data to be predicted, specifying graph data that has a smallest distance from among the plurality of pieces of graph data by using a distance between each label of the plurality of nodes and each of the plurality of pieces of graph data.

4. The prediction program according to claim 3, wherein
the specifying includes
specifying, from among the plurality of pieces of graph data, graph data that has a smallest total value of the distances by using a total value of the distances from the respective labels of the plurality of nodes for each of the plurality of pieces of graph data.

5. The prediction program according to claim 1, wherein
the determining of the prediction result includes
determining, as a prediction result for the graph data to be predicted, a value that corresponds to a label of a specific node included in the similar graph data is determined.

6. An information processing apparatus of using knowledge graph embedding, comprising:
a determination unit configured to determine whether or not graph data to be predicted is data that includes a node link that indicates a relationship between nodes not included in training data used for training of the knowledge graph embedding;
a specifying unit configured to specify, in a case where it is determined that the graph data to be predicted is the data that includes the node link not included in the training data, graph data similar to the graph data to be predicted from the training data based on a result of embedding prediction for a label of a node included in the graph data to be predicted; and
a prediction unit configured to determine a prediction result for the graph data to be predicted based on the specified similar graph data.

7. A prediction method, implemented by a computer, that uses knowledge graph embedding, the prediction method comprising:
determining whether or not graph data to be predicted is data that includes a node link that indicates a relationship between nodes not included in training data used for training of the knowledge graph embedding;
specifying, in a case where it is determined that the graph data to be predicted is the data that includes the node link not included in the training data, graph data similar to the graph data to be predicted from the training data based on a result of embedding prediction for a label of a node included in the graph data to be predicted; and
determining a prediction result for the graph data to be predicted based on the specified similar graph data.
